# EUROPEAN PATENT SPECIFICATION

(11) **EP 3 503 856 B1**
(45) Date of publication and mention of the grant of the patent: **22.07.2026**
(21) Application number: 17844034.3
(22) Date of filing: 22.08.2017
(51) Int. Cl.: A61M 1/00, A61F 13/05

(54) **WOUND DRESSING WITH INFLATABLE STRUCTURES**
WUNDVERBAND MIT AUFBLASBAREN STRUKTUREN
PANSEMENT POUR PLAIES À STRUCTURES GONFLABLES

(30) Priority: 23.08.2016 SE 1651129
(43) Date of publication of application: 03.07.2019
(73) Proprietor: Halao AB, 426 77 Västra Frölunda (SE)
(72) Inventor: ROOS, Håkan, 426 77 Västra Frölunda (SE)
(74) Representative: Westpatent AB
(86) International application number: PCT/SE2017/050842
(87) International publication number: WO 2018/038665

(56) References cited:
- WO-A1-2005/046762
- WO-A1-2014/202560
- WO-A2-2016/015001
- WO-A2-2016/015001
- DE-U1- 202016 000 727
- US-A1- 2005 070 858
- US-A1- 2015 209 493
- US-B2- 8 961 496

## Description

### TECHNICAL FIELD

The present disclosure relates to an organ contact layer. The contact layer is in particular designed to be used in a negative pressure wound therapy of an open wound of the abdominal cavity. The contact layer is intended to be used as a contact layer to be in contact with the intestines and other organs in the abdomen. The present disclosure also relates to a negative pressure wound system as well as the use of a contact layer. An example of a prior art system is described in WO2005/046762.

### BACKGROUND ART

In the surgical technology today it is common practice to treat large wounds with negative pressure wound therapy. Such examples are when operating in the abdominal cavity, after trauma and in patients with abdominal compartment syndrome and/or multi organ failure. By applying a negative pressure the healing process is improved, body fluids may be drained and application of dressings is facilitated. Negative pressure treatment systems for the abdominal cavity may have different design and features but most commonly they comprise the following items:
i. A contact layer, also referred to as fluid transport layer, intended to be close to and in contact with the intestines in an open abdominal cavity.
ii. A wound filler, e.g. a sponge, located in the opening of the abdominal wall and placed on top of the contact layer.
iii. A wound cover membrane, also referred to as an adhesive layer, which is a film layer or sheet attached to the abdominal wall around the edges of the opening in the abdominal wall. The wound cover membrane is used to fixate the wound filler and provide a tight seal around the opening in the abdominal wall. The wound cover layer is made from a tight material so as to function as a seal to create a closed system in the abdominal cavity in order to be able to build up a negative pressure in the abdominal cavity.
iv. A suction device applied to the system and attached to an opening provided in the wound cover membrane in order to create a negative pressure in the abdominal cavity.

Negative pressure wound therapy systems, also commonly referred to as negative pressure wound treatment systems or simply negative pressure therapy systems, for abdominal wounds comprising contact layers, which also may be referred to as fluid transport dressings, are for example disclosed in WO 2014/202 560 and US 2012/136326.

### SUMMARY OF THE INVENTION

One problem associated with applying the negative pressure wound therapy system is to place the contact layer accurately. Another important issue is that the contact layer should be made flexible in order to be kept close to the intestines. It also needs to be possible to wrinkle or fold it in order to fit into the wound and thereafter enable it to be unfolded and deployed between the abdominal wall on one side and the intestines and organs on the other side. When placing the contact layer there is also a problem in keeping it in the desired position once this is achieved. Since the contact layer is thin and lacks rigidity and stability it may thus easily be dislocated when the placing tools or hands are retracted and the sheet will not be kept in place as desired. Both the issue of placement and keeping the desired position are associated with problems in currently available solutions. In general is it desired that the contact layer is placed so that it reaches and covers a large portion, in many cases as much as possible, of the abdominal cavity since its purpose is to aid in draining fluid from all parts of the abdominal cavity. Hence, there is a desire for an improved organ contact layer which facilitates the possibility to accurately position it and keep it in a desired position once this is achieved.

The invention is defined by the appended claims. It is designed to overcome both the issue of placing the contact layer correctly and keeping it in place during treatment. The contact layer as disclosed herein is designed with channels that can be filled with a fluid such as liquid, gel or gas (hereafter referred to as fluid). By filling the channels with fluid it is possible to increase and control the stability and rigidity of the contact layer. Once placed in the abdominal cavity the channels are filled and thus the stability and rigidity of the contact layer can be controlled. By filling the channels, the edges of the contact layer can be pushed further down into the peripheral parts of the abdominal cavity which facilitates positioning and keeps it in place once deployed.

It shall be noted that the contact layer is a completely different feature compared to a wound cover membrane when forming part of a negative pressure therapy system. The contact layer is intended to be in contact with the tissue or wound floor. Its purpose is to protect the tissue and keep the tissues at its location. In addition, the contact layer is preferably designed to be liquid permeable and allow fluid to pass through, e.g. by being perforated. Hence, in order to classify a feature as a contact layer its purpose shall be to be located on top of a wound floor and in contact with the tissue therein during treatment. On the other hand, the wound cover membrane shall be gas tight, and thus also liquid impermeable, in order to enable a negative pressure at the wound site. Furthermore, the wound cover membrane shall be kept at a distance from the wound floor and it should be avoided that the wound cover membrane is in contact with the open wound and get stuck to the tissues at the surface of the wound floor during treatment. If the wound cover membrane is in direct contact with the wound floor it may harm the negative pressure therapy and to keep a distance between the wound cover and the wound floor is important for the negative pressure therapy to work properly.

Hence, the disclosure relates to a contact layer for a negative pressure therapy. What is referred to as contact layer herein may also be referred to as a fluid transport dressing and a negative pressure wound therapy may be referred to a negative pressure wound treatment system. The contact layer has been designed to comprise at least one inflatable channel which is provided with an opening for inflation by fluid. The very same opening, or another opening, could be designed to allow deflation of the channel. In the following, the reasoning will generally be discussed for a contact layer comprising several channels. However, the reasoning will also in general be valid for only one channel unless otherwise specifically stated and the contact layer may thus comprise only one single inflatable channel.

A contact layer is thus a relatively thin, sheet like structure which may be folded and unfolded in order to be located appropriately adjacent to or in contact with a wound floor in a body in order to improve drainage of body fluids form a wound. The channel or channels provided in the contact layer is intended to be able to improve the rigidity and stability of the thin sheet when inflated. As is obvious, the increased stability provided from the pressure of the fluid in the channels will be dependent on the pressure. Hence, the channels shall be designed to be able to maintain an elevated pressure at least for a time during application. In general, the channels are designed to maintain an elevated pressure from the injected fluid until the fluid is controlled to be let out, e.g. by actively deflating a channel through an opening or simply cutting a hole in a channel. However, there is not necessary a desire to keep the rigidity of the contact layer once the contact layer is at its desired location and the negative pressure therapy system is working. Hence, the channels may be provided with a valve arrangement which allows the fluid to slowly be dissipating from the channels. It could even be possible to have one end of the channels open, e.g. at a distal end at or close to an edge of the contact layer, such that the opening is closed by the folding or wrinkling of the contact layer and when the contact layer is deployed during application and positioning will the fluid be allowed to be released through the opening.

The contact layer may comprise a multitude of channels and the specific number of channels is not considered essential. In general, there should be at least four channels present, e.g. two channels to be positioned at each lateral side. In general, there are further channels present in the contact layer and a suitable number may be from 8 to 20 channels. However, it could be possible to design the channels to be meandering, i.e. winding back and forth, such that one channel comprises several portions stretching in different directions and there is thus need for fewer channels and even enough with one single channel. The number of channels may be tried out and there may be further channels than disclosed herein if desired. In general, there should be at least some channels, or portions of a channel, stretching from the centre portion towards the edges of the contact layer. Hence, there is generally a desire to provide channels, or portion of channels, extending in a direction essentially from the centre portion of the contact layer towards its edges. In particular is there a desired to provide channels stretching towards the lateral sides of a human when the contact layer is positioned in the abdominal cavity of a person. Hence, a contact layer as described herein may comprise channels, or portion of channels, designed as oblong channels stretching from the centre portion of the contact layer towards the edges of the contact layer. This design of course also include the possibility that the channels could be designed to stretch from one side to another passing through a centre portion or centre line. The inflatable channels could also be designed as circular, oval or asymmetrical ring channels. They may for example encircle the centre portion, e.g. by being circumferentially located around the centre portion of the contact layer. These channels could be located close to the centre portion and mainly being used to provide stability in the centre portion. However, there could of course also be ring channels present further away from the centre portion, even close to the edges of the contact layer, so as to provide for stability in other portions or regions. There could be a use of both circular channels and essentially linear channels, e.g. stretching from the centre portion towards the edges of the contact layer, in the same contact layer. These geometrical channel designs could also be connected to each other such that a channel includes both ring shaped portions and essentially linear portions.

The channels could have a longitudinal extension between 40 and 800 mm. In general, there is present at least one channel having an extension of at least 200 mm. The inflatable channels may be designed to have a width of between 1 and 100 mm and an average height of between 1 and 40 mm. In general, the channels are intended to have a height and width which both are less than 20 mm.

In case there are several channels present may these be connected by communicating conduits. For example, all channels may be connected such that all channels may be inflated by introducing fluid through one single opening. In another example, the channels may be connected to form two or several groups of channels such that the channels in each group are connected by communicating conduits and each group of channels may be separately inflated. These groups could be designed to comprise relevant channels to be filled together, e.g. could there be a first group formed by channels on a first half portion of the contact layer and a second group of channels formed by channels on a second half portion such that each group of channels on each half portion thus being separately inflated. This may for example be advantageous if a first portion of the contact layer first is located and positioned in a first lateral side of an abdominal cavity, whereby a first group of channels located on this first portion is inflated, where after a second portion of the contact layer is positioned in the second lateral side of an abdominal cavity and a second group of channels located on this second portion is inflated.

Concerning the inflation and design of the channels could of course also all channels be separated to be separately filled through an opening in each channel. However, if there are many channels present this may be time consuming.

In general, when it shall be distinguished between what is considered to be one channel or if it may be considered as several channels is if there is an essential and drastic change of the dimensions in the cross sectional area of the space forming the confined space into which fluid is introduced. For example, if there is a slight increase in the height or width of the channel along its length it is considered to be one single channel while if the channel is opening up into a chamber having a width or height which considerably extends the medium height or width of the channel such a chamber is in general not considered to form part of the channel and other channels connected to such a chamber are thus considered to be separate channels.

The channels, or portions of channels forming a meandering structure stretching in essentially parallel directions, are located such that a distance between two adjacent channels or portions is between 5 and 250 mm, in general are two adjacent channels located such that they along some portions of their extension is between 50 and 200 mm . The channels may have different directions and the distance between them may therefore vary along the extension of the channels.

The contact layer could be designed to comprise one or several sheets of material. The sheets may comprise or be entirely made of plastics material. A suitable plastic material is for example polyurethane and the sheets could be made of polyurethane or a mixture of polyurethane with other polymers. A plastic material used for the sheets could of course also comprise additives if desired to achieve desired properties of the sheet material. The contact layer could comprise a single layer with a single or a plurality of inflatable channels welded to this layer. The channels could be positioned on either side of the sheet or on both sides. In case there is only one single channel it is most natural to locate it on one side but it could of course be passing through the sheet to be on both sides. If the contact layer comprises at least two sheets it could comprise a top sheet and a bottom sheet and one or a plurality of inflatable channels located there between. The channels could for example be made by using a third intermediate sheet interposed there between which is welded to both layers while forming channels. However, if only the top sheet and bottom sheet are used could they be welded together such that one or several channels are created in between the sheets. If further sheets are used there may be channels formed on different levels. The total length of the channel or channels should be at least longer than 0,5 metres, preferably longer than 1 metre and in particular longer than 1,5 metres. The contact layer is preferably designed to have an area of at least 0,05 square metres, in general having an area of at least 0,10 square metres in order to enable a desired covering of the abdominal cavity.

The contact layer could be sterilized in order to be suitable to be used in a negative wound pressure therapy where it is intended to be in contact with organs and/or other parts of a body. Hence, the contact layer is intended to be adapted for use with wounds and form part of a negative pressure therapy system and is suitable for abdominal wounds. Both in the case of a single sheet or a top and a bottom sheet these may be fenestrated between the inflatable channels to make said layers liquid permeable.

The contact layer as described herein could comprise sealed chambers in addition to the inflatable chambers. The feature of including sealed chambers in a contact layer is for example described in WO 2014/202 560, which refers to the contact layer as a fluid transport dressing. The inclusion of sealed chambers filled with a fluid in the contact layer may improve the permeability of the contact layer and thus improve the fluid transport properties of the contact layer as well as providing a cushioning effect of the contact layer. In addition, since the sealed chambers shall be filled with a fluid could also the inflatable channels easily be pre-filled with fluid when the sealed chambers are filled at manufacturing. This of course implies that there is desired to include some kind of one way valve mechanism in the opening for filling and inflating the inflatable channels such that the fluid filled into the channel will not escape. The inflatable channels are preferably filled only to such an extent that the contact layer may be easily folded and wrinkled in order to be inserted into the wound where it is intended to be used in forming part of a negative pressure therapy system. When the contact layer already is inserted to the open wound the inflatable channel or channels may be further filled and inflated in order to provide improved stability to the contact layer and facilitate positioning of the contact layer. Even though it has been explained in this paragraph that the feature of pre-filling of fluid into the inflatable channels is particularly suitable when it may be made at the same time as the filling of a fluid into sealed chambers may of course the inflatable channels be prefilled even though there are no sealed chambers present. If the channels are prefilled with a fluid a smaller amount of fluid would be needed to fill up and pressurize the inflatable channels. It could for example be desired to be able to fill and pressurize several channels with one single loaded syringe and if the channels are prefilled, more channels may be inflated to a desired level by a single load of fluid from the syringe.

The contact layer is preferably liquid permeable. This may for example be made by perforating a layer made from a liquid impermeable sheet material. The size and shape of the perforations may vary according to a desired through flow or other relevant parameters. The perforations may be made all over the sheet or at selected locations or portions where it is desired to have a through flow. The contact layer could of course also be made from a sheet material which is liquid permeable by itself. Since there shall be inflatable channels in the contact layer, the walls of the channels must of course be liquid tight in order to be able to inflate and pressurize the channels

The disclosure further relates to a negative pressure therapy system kit, e.g. to be used in the abdominal cavity. The kit comprises a contact layer as herein described which is adapted to be applied onto a wound floor, e.g. intestines of an abdominal wound. The kit further comprises a wound filler adapted to be placed on top of the contact layer when it has been located in the wound. The kit also comprises a wound cover membrane intended to be attached at or close to walls of the wound, e.g. to the skin surrounding the wound. The wound filler, which may be a sponge or other elastic porous material allowing fluid to be transported there through and creating a distance between the contact layer and the wound cover membrane, is adapted to be located in a space between said wound floor and the skin and could for example be located between a wound floor and an abdominal wall in case it is used in the abdominal cavity. Hence, the contact layer as disclosed herein is suitably used as a contact sheet to be located between wound floor and the abdominal wall.

The contact layer as disclosed herein could be applied by using a method (not forming part of the invention as claimed) in which the contact layer, which first is folded or wrinkled, is introduced to the location where it shall be positioned and there after unfolded and located at its desired location whereby the channels are inflated by a fluid. This method could for example be used in a method of treating an abdominal wound or open abdomen with negative pressure therapy wherein an abdominal cavity is accessible for therapy. Such a method could be described to comprise the following steps:
1. Applying a contact layer inside the abdominal cavity.
2. Filling at least some of the channels which are comprised in the portion of the contact layer to be positioned first, e.g. channels comprised in the central portion of said contact layer in order to enable easy centring in the opening of the abdominal wound.
3. Subsequent filling of channels in the portions of said contact layer which are to be positioned thereafter, e.g. parts of the contact layer intended to be placed in the peripheral and deeper parts of the abdominal wound.
4. Applying a wound filler inside said abdominal wound and on top of the contact layer
5. Covering said abdominal wound with a wound cover membrane. The wound cover membrane should preferably be attached to the circumference of the wound and form a tight seal so as to enable a negative pressure to be achieved in the abdominal cavity.
6. Assuring the presence of an opening in a part of said cover film over said wound filler, e.g. by cutting a hole in the wound cover membrane or locating a prefabricated opening in the previous step, at a desired position, e.g. in a centre part of the wound cover membrane covering the open abdominal wound. The opening shall be designed to fit in with a negative pressure source to be connected to the abdominal cavity.
7. Applying a negative pressure from the negative pressure source, e.g. a pump, to said opening in the wound cover membrane for creating a negative pressure in said abdominal cavity.

In the following, the invention will be explained with reference to some examples disclosing different embodiments of how the invention described above may be realized.

### BRIEF DESCRIPTION OF THE DRAWINGS

The present disclosure will be explained below by means of non-limiting examples with reference to the accompanying drawings, in which:
- Fig. 1: shows a cross sectional view of a negative pressure wound therapy system comprising a contact layer;
- Fig. 2: shows an isometric view of a contact layer comprising inflatable channels;
- Fig. 3: shows different designs of inflatable channels in a contact layer;
- Fig. 4: shows an isometric view of a contact layer comprising inflatable channels and closed chambers;
- Fig. 5: shows a design of inflatable channels and closed chambers in a contact layer; and
- Fig. 6: shows a contact layer provided with slits.

### DETAILED DESCRIPTION OF THE DRAWINGS

The disclosure will, in the following, be exemplified by embodiments. It is to be understood, however, that the embodiments are included in order to explain principles of the invention and not to limit the scope of the invention defined by the appended claims.

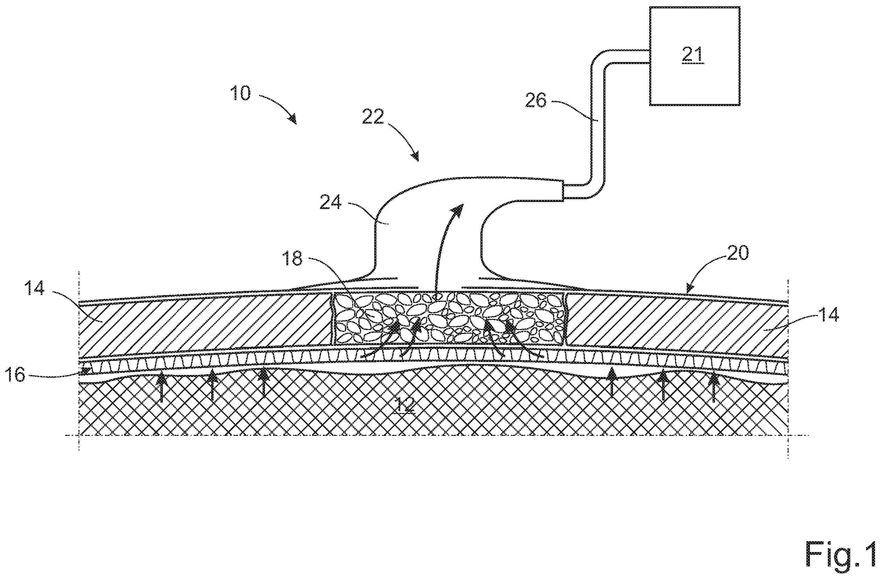

Figure 1 illustrates a negative pressure wound therapy system 10. In figure 1, the system 10 is exemplified as a negative pressure wound therapy system for use in the abdomen. As such, the system 10 is adapted for use with abdominal wounds and illustrates the wound floor 12 as well as an abdominal wall 14 of the abdominal wound / open abdomen. The intestines in the bottom of the abdominal cavity can be regarded as the wound floor 12.

The negative pressure wound therapy system 10 comprises an organ contact layer 16 at least a portion of which is adapted to be located between the wound floor 12 and the abdominal wall 14. Furthermore, the system 10 comprises a wound filler 18 adapted to provide fluid transport between the organ contact layer 16 and a negative pressure source 21. Purely by way of example, the wound filler 18 may comprise a foam material, for instance an open-celled foam material. As a non-limiting example, the wound filler 18 may comprise a flexible open-celled foam material, such as a sponge material. Examples of suitable foam materials include, without limitation, materials comprising polyurethane, polyester, polyether or polyvinyl alcohol or combinations thereof. As a non-limiting example, the foam may be a hydrophobic polymer foam. As another non-limiting example, the wound filler 18 may comprise gauze.

Moreover, although purely by way of example, the negative pressure source 21 may comprise a negative pressure pump which may be referred to as a vacuum pump. Purely by way of example, the negative pressure source 21 may be adapted to provide a negative pressure, the absolute value of which is greater than or equal to a threshold value. Generally, the threshold value in such embodiments is at least 20 mmHg. In some embodiments, the negative pressure source 21 is adapted to provide negative pressure at one fixed threshold value.

The negative pressure wound therapy system 10 further comprises a wound cover membrane 20. The wound cover membrane is generally adapted to be attached to the skin surrounding the wound. Purely by way of example, the wound cover membrane 20 may comprise a wound cover film. The wound cover membrane 20 may preferably be attached to the skin surrounding the abdominal wound, for instance by means of an adhesive. Examples of adhesives that may be used include, but are not limited to, acrylic adhesives and/or silicone gel adhesives. In some embodiments, the adhesive or adhesives is/are already incorporated as part of the wound cover film. In some embodiments, the adhesive or adhesives is/are applied to the wound cover member during use. Purely by way of example, the adhesive sold under the trademark Mepiseal^{®} by Molnlycke Healthcare AB may be used for attaching the wound cover member to the skin surrounding the wound membrane during use.

Figure 1 further illustrates that the negative pressure wound therapy system 10 comprises a fluid communication assembly 22 adapted to provide a fluid communication between the negative pressure source 21 and the wound cover membrane 20. The fluid communication assembly 22 may for instance comprise a suction device 24 and a conduit assembly 26 comprising one or more conduits.

Arrows in figure 1 indicate how liquid may travel from the abdominal wound towards the negative pressure source 21 via the organ contact layer 16, the wound filler 18 and the fluid communication assembly 22, respectively.

The negative pressure wound therapy system 10 described in figure 1 thus discloses one of many examples of a negative pressure wound dressing in which the organ contact layer 16, which is to be described in more detail below, may be applied.

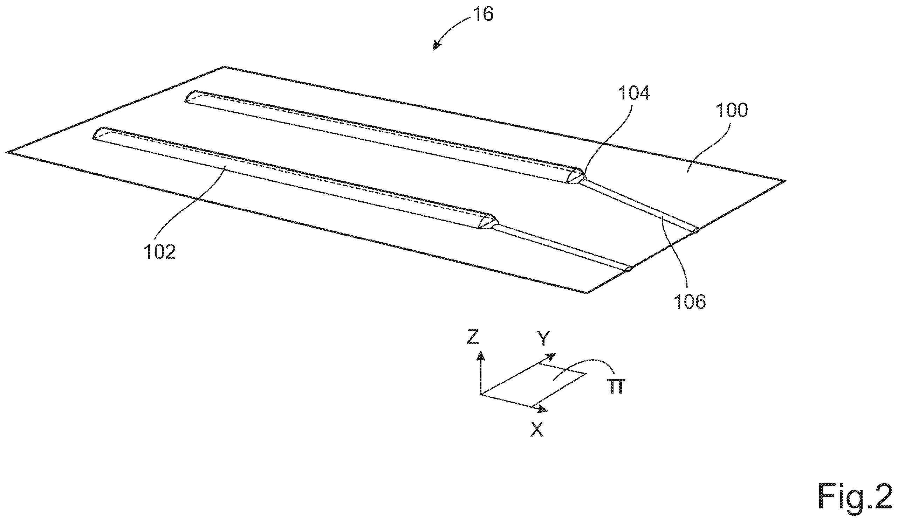

Figure 2 illustrates a portion of an organ contact layer 16 according to an embodiment of the present invention. The organ contact layer 16 comprises one or a plurality of inflatable channels 102 which have been inflated with a fluid. The purpose of these channels is to provide rigidity and stability to the organ contact layer when the channels 102 are filled with a fluid. The rigidity and stabilizing effect depends among other things on the level or pressure of the filling fluid. By determining the level of rigidity and stability of the organ contact layer it also makes it possible to shape it accurately and position the organ contact layer optimally.

The organ contact layer 16 has a planar extension in a first plane π defined by the X- and Y-axis. In this case is the organ contact layer exemplified by a thin sheet 100. The thin sheet 100 is provided with a plurality of inflatable channels 102 (only one channel disclosed in the portion shown in figure 2). The channels 102 are elongated and extend in its length direction on the surface of the thin sheet 100 in the first plane π. As is obvious from figure 2, the channel 102 also has a height such that it protrudes in a direction V perpendicular to the plane π. The sheet 100 in figure 2 is shown when the channel 102 is inflated with a fluid. When the channel 102 is not inflated, the channel is essentially flat and levelled with the thin sheet 100. In this case it is shown that the base of the channel is essentially levelled with the thin sheet 100 and protrudes upwards, in the positive V-direction. However, the channel 102 may also be designed such that its base is levelled with the thin sheet 100 and protrudes downwards, in the negative V-direction. The channels could also be designed such that the very same channel protrudes in both the negative and positive V-direction, i.e. a channel is protruding from the plane π on both the upper and lower side of the sheet 100. In a sheet 100 comprising a multitude of channels 102 may these channels be designed such that all of them are designed to protrude upwards, downwards or in both directions. Alternatively, the sheet 100 may comprise channels 102 protruding differently in the V-direction, e.g. some protruding upwards and others downwards.

Each one of the channels 102 is provided with at least one opening 104 in order to be inflated. The openings 104 may be connected via linking conduits 106 to a source (not shown) for filling and inflating of the channels 102. As a source for filling the channels 102, linking conduits 106 or chambers 110 may be used (purely as an example) a syringe filled with fluid. The linking conduit for filling (which may have a diameter/width/height that is smaller, equal to or larger than the channels) may be directly connected to the channels or to a common chamber or several connected channels. For example, all or a group of channels 102 may have their linking conduits 106 connected to a common chamber or some kind of manifold such that each one of the channels 102 is directly connected to the common chamber. This chamber is thus adapted to be connected to a fluid source in order to be filled with a fluid and delivering the fluid to the respective channels 102 via their linking conduits 106 or by being directly in contact with the opening 104 of the channels. Alternatively, all or some of the channels 102 may be connected in series such that the filling fluid will pass through one or more channels 102 in order to fill up further channels, which shall be inflated from the source. In this figure, i.e. figure 2, the channel 102 is shown as a channel having a single inlet opening 104 connected to a linking conduit 106. In those cases the channels will have a through flow in order to fill up further channels, these channels will obviously have two or more openings having at least one linking conduit 106 connected to another channel 102. The openings 104 and/or the linking conduits 106 may be provided with a one-way valve arrangement such that once a channel 102 is filled it will automatically stay filled until the one way valve is punctuated or in some way disabled. In case there are several inflatable channels 102 connected in series to be filled, the arrangement may be designed such that there is only a one-way valve located in the first channel to be filled which thus will function to keep all the channels connected in series to stay filled when the connected channels are filled. When a common chamber or manifold is used and connected to several channels, a similar valve arrangement may be used for the opening inlet to the common chamber or manifold such that the filling fluid may not escape the reverse way through the common chamber and the channels connected to the common chamber will also stay filled. One or several independent or series of interconnected linking conduits, channels and chambers may be used in the contact layer. Each of the series or parts of series of channels may have separate one-way valves. Even though it generally has been mentioned a multitude of channels 102 above could they be replaced for one single channel to be used.

The channels 102 may vary in size from being about 40 millimetres to 800 millimetres long and having a width of 1 to 100 millimetres. The height is in a range from 1 to 40 millimetres. Most commonly the channels vary in size from being about 40 millimetres to 400 millimetres long and having a width of 3 to 25 millimetres and a height in a range from 3 to 25 millimetres. It is generally considered that the height and width are essentially of the same dimensions or that the width may be larger than the height even though this is not necessary. The height and/or the width may vary along the longitudinal extension or be the same. In case the height and width are of essentially the same dimensions the channels will assume an essentially circular cross sectional area, i.e. having an essentially cylindrical shape, when inflated with the filling fluid. In case the width is greater than the height will the cross sectional area be more oval. The geometrical shape may also have other geometries all though these geometrical shapes described above will be natural to form when the channels are filled and pressurized with fluid. The volume of a channel is generally thought to be in the range from 0,020 ml to 30 ml. A channel having a volume of 0,02 ml corresponds essentially to a cylindrical chamber having a diameter of 1 mm and being 25 millimetres long while 20 ml corresponds to a cylindrical channel having a diameter of 10 mm and a length of about 250 millimetres. A cylindrical shaped channel having a diameter of around 5 millimetres and being 100 millimetres long will thus have a volume of about 2 ml. The channels may be straight or curved. The thin sheet 100 may have a thickness within the range of 0.01 - 1 mm. The thickness of the sheet is generally thought to be 0.01-0.2 mm. The walls of the chambers may have the same thickness. In practice, the walls of the channels 102 are preferably made from the same kind of material as the thin sheet 100. The thin sheet 100 and the chambers 102 may be made of plastics which may comprise polyurethane.

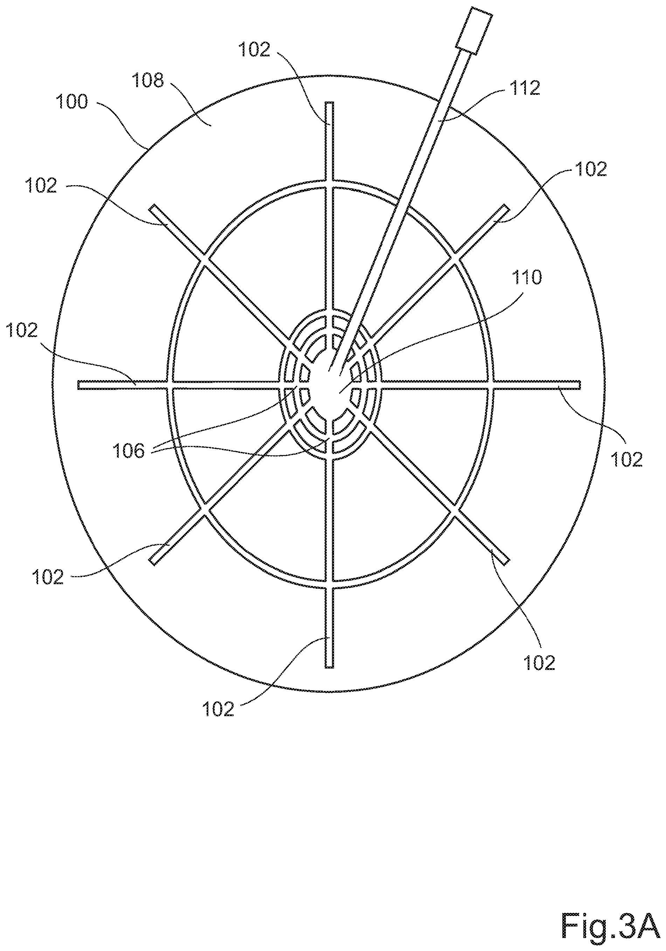

In figures 3a to 3f different designs of the inflatable channel or channels 102 in the organ contact layer 16 are shown. The organ contact layer 16 is in general designed to have a circular or essentially circular shape. However, the shape could be altered, e.g. oval or asymmetrical, while still working perfectly well for the present invention and it is obvious for the skilled person to change the geometrical shape of the sheet 102 as desired. All the designs in figures 3a to 3f are intended to address the issue of placing the organ contact layer adequately in the flanks and peripheral parts of the abdominal cavity and to secure this placement after deployment. The concept of all designs 3a to 3f are a system of channels 102, distribution chambers 110 and linking conduits 106 in the organ contact layer 16. The orientation of the channels 102, conduits 106 and chambers 110 are placed to enable filling of the channels 16 and thus increase the stability of the organ contact layer 16 in the direction desired. Designs 3b, 3d, 3e and 3f shows designs where the channels 102 are grouped in separate systems to enable sequential filling of different parts of the organ contact layer 16. All the designs in fig 3a to 3f are possible to perforate to make the organ contact layer liquid permeable.

In figure 3a is disclosed a first design of the layout of the organ contact layer 16 in which eight channels 102 are located on a circular sheet 100. The channels 102 are stretching in a direction radially outwards from a distribution chamber 110 in the centre portion 108. The distribution chamber 110 comprises an inner circular chamber and two outer, ring shaped chambers located concentrically around the circular chamber. The chambers are connected to each other via four linking conduits 106, two linking conduits connects the circular chamber with the inner ring chamber and two other linking conduits connects the inner ring shaped chamber with the outer ring shaped chamber. The channels 102 are located equidistant and symmetrically around the centre portion 108 of the organ contact layer 16. Fluid may be injected into the circular chamber 110 by the use of an injection conduit 112 connected to the circular chamber 110. The injected fluid is distributed from the chambers 110 to the respective channels 102 via a respective opening 104 in each channel 102. The fluid to the longitudinally stretching channels will be distributed directly from the circular chamber, the fluid to the two transversally stretching channels will be distributed from the inner ring shaped chamber and the fluid to the 4 diagonally stretching channels will be distributed from the outer ring shaped chamber. The fluid is either directly distributed from a chamber 110 to a channel 102 or via linking conduits 106.

The distinction between what is referred to as a channel 102, a linking conduit 106 and a chamber 110 is mainly defined by its function. All these features represent spaces which are intended to be filled up with a fluid entering the system from the injection conduit 112. However, the inflatable features have been defined herein with respect to their intended main function. Features referred to as a channel 102 are features that are designed to provide stability and rigidity to the organ contact layer 16. Elements referred to as linking conduits 106 are features that are designed to enable transport of the fluid between channels 102, between a channel 102 and a chamber 110 or between chambers 110. Elements referred to as chambers 110 are features that are designed to receive fluid and distribute the fluid to a multitude of channels 102, either by being in direct contact with an opening 104 in a connected channel 102 or via a linking conduit 106. A space may have one, two or all three of these functions, but are in this context referred to by their main function.

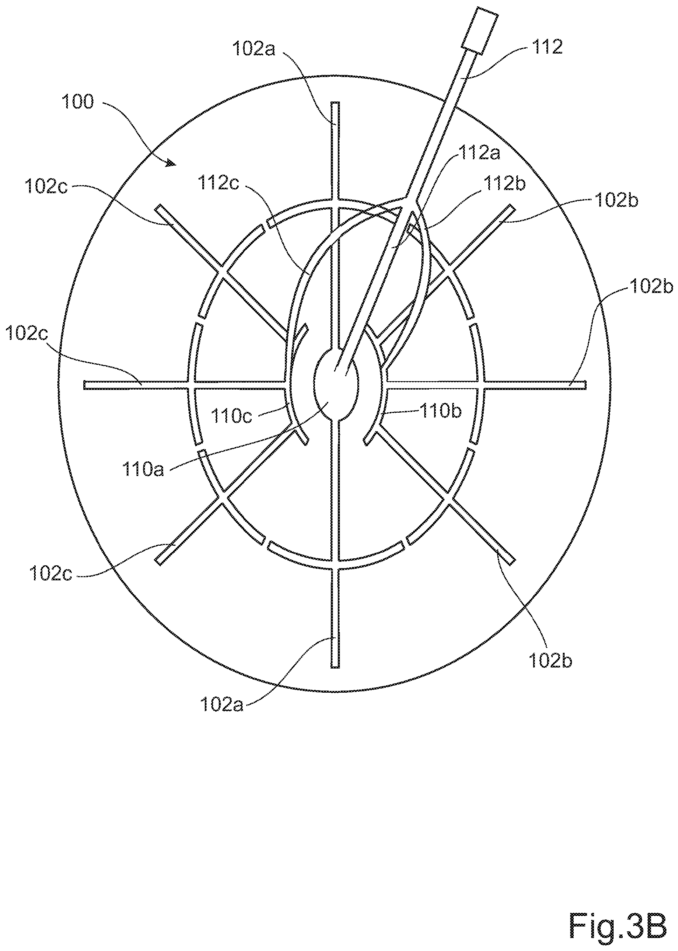

In figure 3b the layout is essentially the same but in this case there are 3 separate chambers 110a, 110b, 110c to which the fluid is injected. In addition, each of the longitudinal channels have been provided with an arc shaped segment which crosses the longitudinal channels about 1/3 of their lengths from the centre in a direction perpendicular to the extension direction of the longitudinal channels. The fluid is injected via three separate injection conduits 112a, 112b, 112c. The first chamber 110a is connected to circular channels 102a close to the centre portion 108 and longitudinal channels 102a which are intended to stretch along the length of a body when the organ contact layer 16 is positioned in the abdominal cavity of a person. The second chamber 110b is connected to three channels 102b which are stretching towards a first lateral side of a person when the organ contact layer 16 is placed in the abdominal cavity. The third chamber 110c is connected to the remaining three channels 102c stretching towards the other, second lateral side of a person when the organ contact layer 16 is placed in the abdominal cavity. Grouping of the channels 102a, b, c in this way makes it possible to first provide rigidity in the central portion of the organ contact layer 16 by filling the first chamber 110a and its connected channels 102a and thus stabilize this portion in a desired position. Thereafter, the first lateral side of the sheet, comprising the second group of channels 102b, may be unfolded and deployed in order to be provisionally located as desired before the second chamber 110b is filled and the fluid distributed to the second group of channels 102b. The first lateral side will thus have improved rigidity and stability facilitating deployment and securing placement. Finally, essentially the same procedure is performed with the other, second lateral side by placing the sheet and filling the third chamber 110c in order to provide an increased stability and rigidity by the third group of channels 102c.

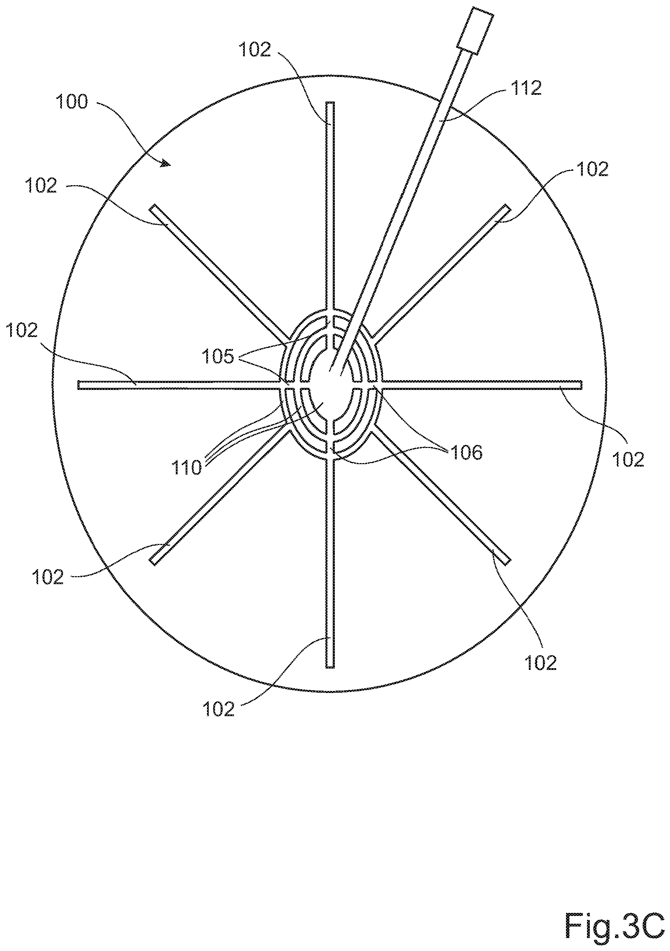

In figure 3c a similar system as described in figure 3a is shown wherein the fluid is distributed to all channels 102 via a single injection conduit 112. However, the system has been provided with four further channels and the orientation of the channels 102 have been designed to extend more in the lateral direction, i.e. to extend in a direction from the central portion 108 towards the sides of person. By lateral direction is meant a direction perpendicular to the longitudinal extension of a person when the organ contact layer 16 is located as intended in the abdominal cavity of a person. This configuration is due to the problems with being able to keep the sides in the desired location when placing the organ contact layer 16 in the abdominal cavity. This design is intended to address particularly the issue of placing the organ contact layer adequately in the flanks and peripheral parts of the abdominal cavity and to secure this placement after deployment. Hence, it is in general most important to provide rigidity in the lateral direction. These basic ideas for designing the layout of the channels in figure 3c could of course also be used for changing the geometrical configuration and extension direction of the channels 102 in figure 3a and channels 102a, 102b, 102c in the arrangement in figure 3b to have channels with more transversal extension.

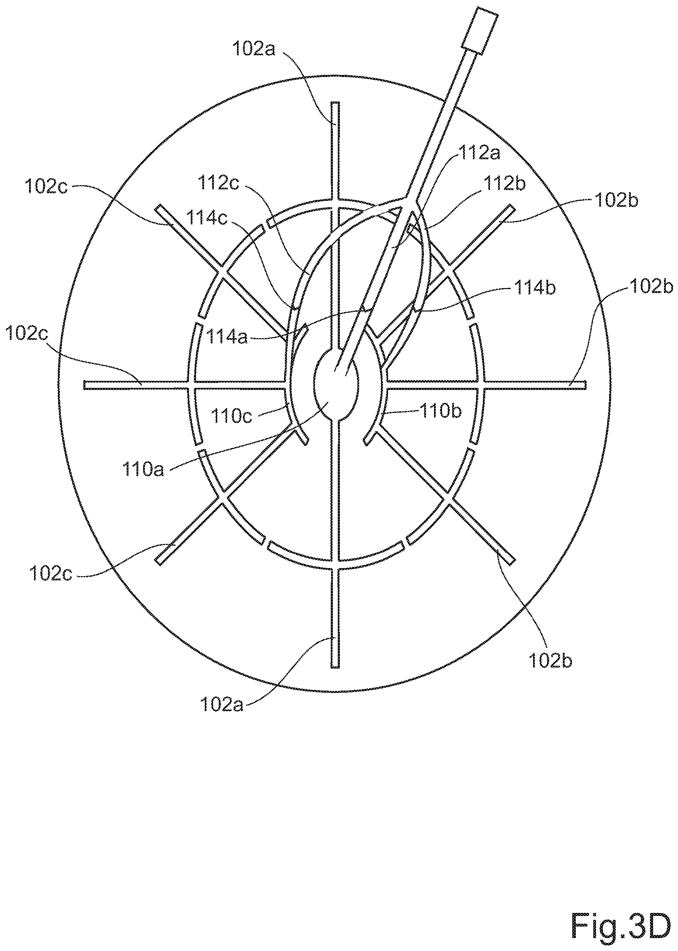

In figure 3d an arrangement similar to the one described in figure 3b is shown but with the difference that each of the injection conduits 112a, 112b, 112c have been provided with a respective one-way valve 114a, 114b, 114c. These valves will thus prevent the fluid in the injection conduits 112a, 112b, 112c to flow in the reverse direction and hence enables pressure to be maintained in the chambers 110a, 110b, 110c and its associated group of channels 102a, 102b, 102c. Such valves could of course also be included in the arrangements described in figures 3a to 3c.

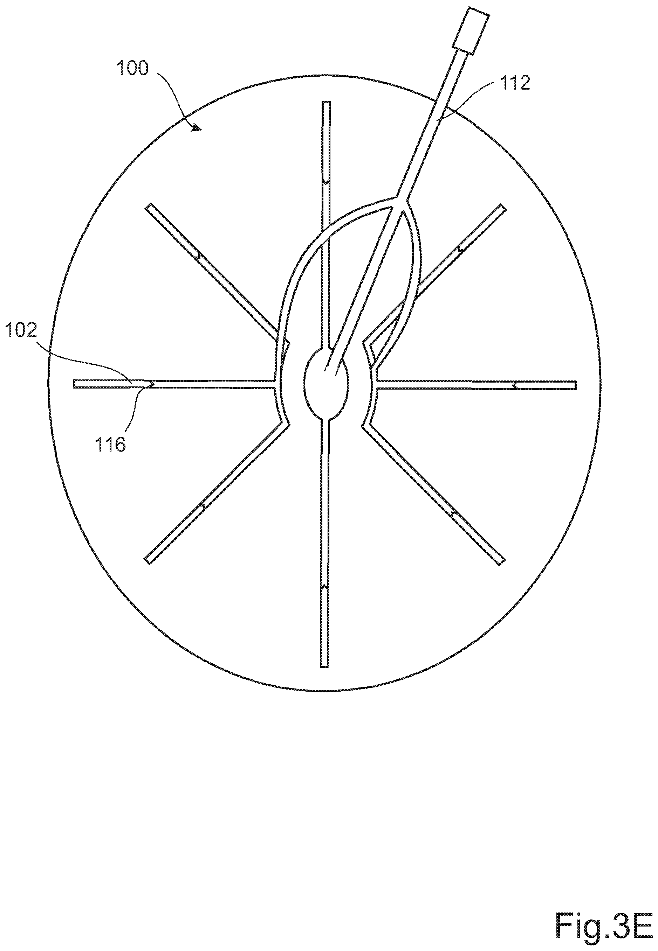

In figure 3e another arrangement is shown which reminds of the layout in figures 3b and 3d by having groups of channels 102a, 102b 102c which may be filled separately. In this arrangement the elongated channels have been provided with low burst pressure valves 116 such that the complete channels will not be filled unless the injected fluid rises above a certain pressure. Hence, by controlling the pressure at injection it will be possible to select if the complete channels of the same group shall be filled or if it is desired only a portion shall be filled. The same arrangement with valves having low burst pressures can be use between sections in the organ contact layer as an alternative to sections that are totally separated. The same arrangement with low-pressure burst valves could of course be used in all designs 3a to 3d.

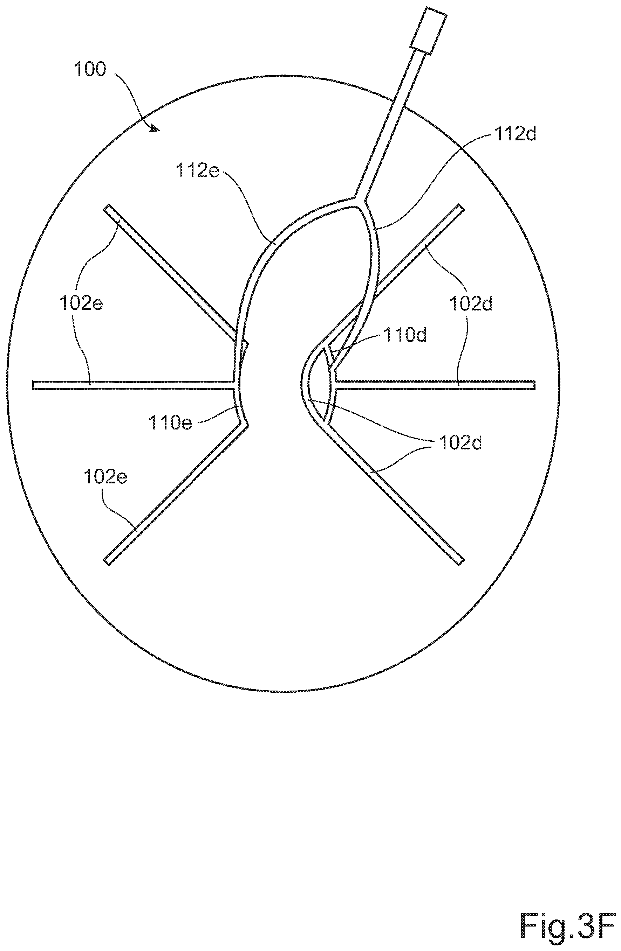

In figure 3f still another arrangement is disclosed in which essentially all channels 102 are designed to be in the lateral direction, i.e. in a direction perpendicular to the length direction of a person when the contact layer is placed in the abdominal cavity as intended. The channels have been divided in two groups, 102d and 102e. To the first group of channels 102d, which essentially comprises channels extending from the centre of the contact layer 16 towards a first lateral side, is also included a centrally positioned semi-circular channel. This arrangement thus provides for inflating the central portion and one side at the same time to be stabilized. When the contact layer 16 has been provisionally adjusted in the central region and at one side these channels 102d could be inflated. Thereafter may the other lateral side comprising channels 102e be provisionally located where after also channels 10e' may be inflated to stabilize the fluid transport sheet16. It shall be noted that even though the channels of both groups are essentially aligned in a transversal direction (except for the central semi-circular channel) is the configuration of the channels 102a', 102b' in this example non-symmetrical and the channels have different lengths with no particular symmetry. This is disclosed in this way in order to make it clear that there is no need to make the channels disclosed herein in a symmetrical pattern. There may be advantages in providing an asymmetrical pattern due to the asymmetrical shape of a human body and the unpredictable exact configuration of a human body. It may for example be advantageous to have the different sides being different such that the configuration of the contact layers channels, and thus rigid zones, will change as the transport dressing is rotated 180 degrees. For example, one side may be wider (having longer channels for one lateral side). Hence, the channel patterns in the embodiments disclosed in figures 3a to 3f may also be designed to be less symmetric. In addition, it may be desired to have different cross sectional size of different channels.

One should note that only the main characteristics have been discussed concerning these examples of an organ contact layer. For example, all the above described organ contact layer may be made liquid permeable by providing slits or holes in the sheet. There may of course be further channels included in the contact layer and the layout and design could also be different. In the examples above is only disclosed that one channel, all channels, 2 groups or 3 or a larger number of groups of channels will be filled at the same time. It could of course be possible to group the channels as desired and use more groups or even have each channel being independently controlled to be filled. Hence, all these embodiments only show a few examples of possible configurations and arrangement of inflatable channels in an organ contact layer.

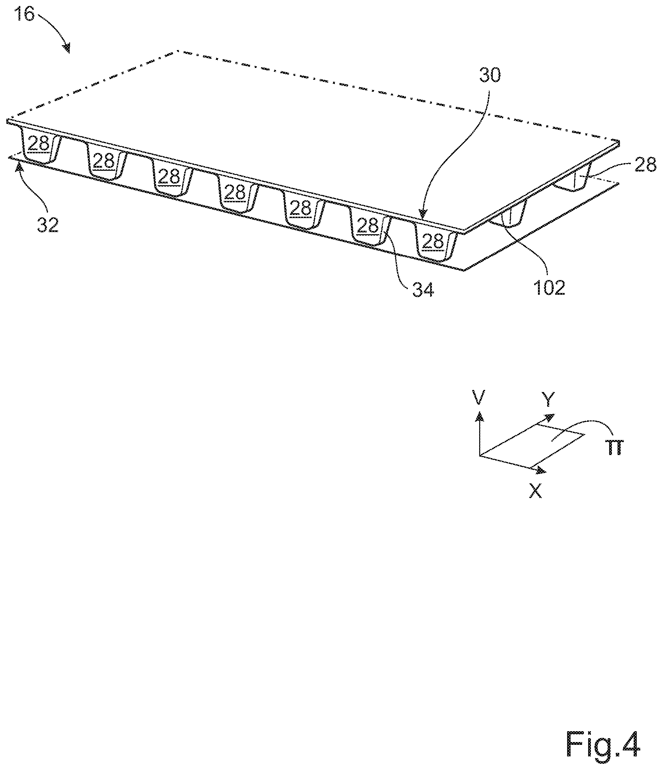

All the above designs can be achieved by using a single thin sheet 100 or a top sheet and a bottom sheet and having the channels 102 interposed between these sheets. All the above designs could also be used in a single sheet with the channels 102 welded to this sheet 100. This design could for example be used for integrating the channels 100 in an organ contact layer as disclosed in WO 2014/202560, which hereby is incorporated by reference, and will be described next with reference to figure 4.

In figure 4 is disclosed an alternative design of the organ contact layer 16 in which the single sheet 100 described in figure 2 has been replaced with a top sheet 30 and a bottom sheet 32. As described for figure 2, the organ contact layer 16 may have a planar extension in a first plane π and the organ contact layer may also extend in a vertical direction V that is perpendicular to the extension of the first plane π. At least one, alternatively both, of the top and bottom sheets 30, 32 may be of a plastics material which may comprise polyurethane. In this design, the organ contact layer 16 has been provided with sealed chambers 28 in addition to the inflatable chambers 102. The sealed chambers 28 and the inflatable chambers 102 are located between the top and bottom sheets 30, 32. The sealed chambers 28, and the inflatable chambers 102, are at least partially delimited by an intermediate sheet 34 that extends at least partially between the top and bottom sheets 30, 32. The intermediate sheet 34 may be deformed, for instance pleated, and thereafter attached to at least one of the top and bottom sheets 30, 32 such that the sealed chambers 28 and/or the inflatable channels 102 are formed. The intermediate sheet 34 may be attached to both the top and bottom sheets 30, 32 such that a continuous organ contact layer 16 is obtained.

Purely by way of example, each one of the sealed chambers encloses a volume of at least 0,010 ml. Alternatively, each one of the sealed chambers encloses a volume of at least 0,050 ml. As another non-limiting option, each one of the sealed chambers encloses a volume of at least 0,080 ml.

In other non-limiting examples of a sealed chamber, the sealed chamber may have a substantially cylindrical shape with a height, i.e. an extension in a direction perpendicular to the planar extension of the top sheet 30 and/or the bottom sheet 32, and a diameter in the planar extension. Purely by way of example, the height of a sealed chamber may be within the range of 0.3 - 0.7 of the diameter of the sealed chamber. The channels 102 may be of the dimensions earlier discussed and the sealed chambers 28 and the channels 102 may be adapted to each other such that they have equal height.

Purely by way of example, the sealed chamber may have a diameter that is one of the following: 3, 6, 9 or 25 mm. Assuming, as a non-limiting example, that the height of the sealed chamber is approximately half the diameter of the sealed chamber, the volume of the sealed chamber is approximately 0,010 ml for a sealed chamber with a diameter of 3 mm, approximately 0,080 ml for a sealed chamber with a diameter of 6 mm, approximately 0,28 ml for a sealed chamber with a diameter of 9 mm and approximately 6,0 ml for a sealed chamber with a diameter of 25 mm.

As a non-limiting example, the intermediate sheet 34 may be of a plastics material which may comprise polyurethane. Instead of, or in addition to polyurethane, at least one, alternatively two or more, of the top, bottom and intermediate sheets 30, 32, 34 may comprise at least one of the following materials: other types of urethanes, silicone, transparent hydrocolloid, PVC, hydrogel, copolyester, polyethylene, TPS (thermoplastic elastomers based on styrene) or TPO (thermoplastic olefins) i.e. blends of polyethylene and polypropylene.

Purely by way of example, each one of the top and bottom sheets 30, 32 as well as the intermediate sheet 34 may have a thickness within the range of 0.01 - 1 mm.

In a specific embodiment could the contact layer 16 be manufactured such that it essentially only has what appears to be sealed chambers 28 when the dressing 16 is manufactured. There are however a few chambers 28' which has been adapted with an inlet opening 104, either being void or having a one-way valve at the inlet opening. The contact layer could be manufactured such that a group of sealed chambers 28 have been prepared to release the attachment of intermediate walls when subjected to an elevated pressure. This design could thus enable a contact layer 16 which appears only to have a multitude of small sealed chambers 28 to form larger channels 102 by injecting a fluid into these special chambers 28' which is adjacent to a group of sealed chambers which are designed such that their intermediate walls will collapse and they will form a large channel together when fluid is injected. The collapse may for example be made, if a design using a top sheet 30, bottom sheet 32 and intermediate sheet 34, by designing the intermediate sheet 34 to have a weaker attachment to the top sheet 30 and/or bottom sheet 32 at predefined locations such that it will detach in a controlled manner and a group of sealed chambers will form an inflatable channel.)

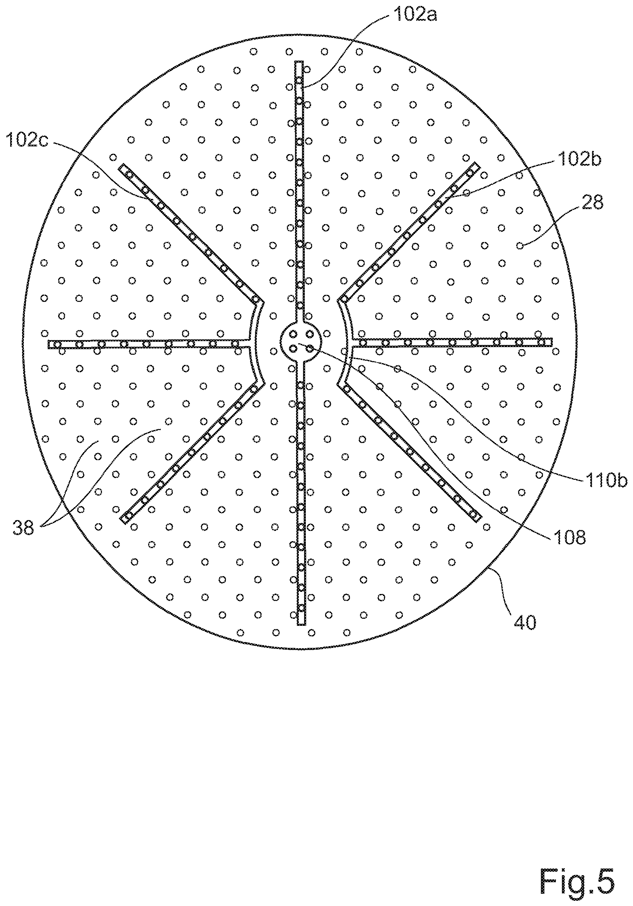

Fig. 5 illustrates a top view of how a contact layer 16 could be designed having sealed chambers 28 and inflatable chambers 102a, 102b, 102c. The layout may for example be used for the contact layer 16 of which a portion is disclosed in figure 4. In practice, there should probably be more sealed chambers than what is disclosed in this figure. In case the transport dressing is made as disclosed in figure 4 can it be seen that fluid conduits 38 will be formed between rows of sealed chambers 28 and/or inflatable channels 102a, 102b, 102c. These channels 102a, 102b, 102c could either be designed to be channels when manufactured or be formed by interconnecting a row of specifically designed sealed chambers 28' which are designed to rupture their side walls when subjected to an increased pressure and thereby interconnect with each other and form a large inflatable channel 102 together.

If this layout described in figure 5 is used for the dressing described in figure 4 it further indicates a plurality of fluid conduits 38. The sealed chambers 28 are arranged such that at least a plurality of the fluid conduits 38 extend from the periphery 40 of the contact layer 16 towards the centre 108 thereof. However, in other embodiments of the contact layer 16, the sealed chambers 28 may be arranged in other ways. For example, the sealed chambers 28 may be spaced evenly throughout the contact layer.

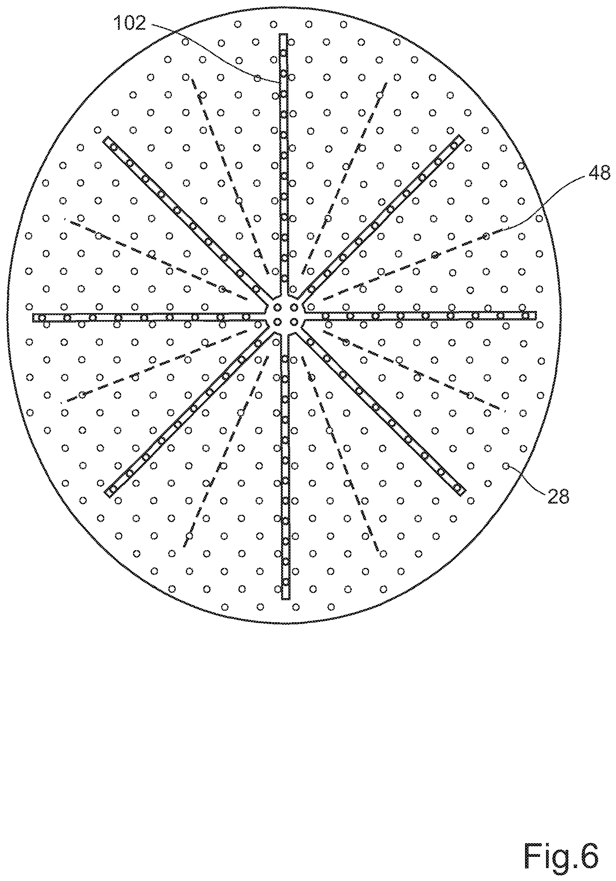

In figure 6 is disclosed an example in which the organ contact layer 16 has been provided with slits 48 in order to make permeable to liquids. In case a double sheet structure is used as disclosed in figure 4 should of course both sheets be provided with slits 48 in order to make the organ contact layer readily permeable to fluids.

## Claims

1. A negative pressure wound treatment system (10) for an open wound of the abdominal cavity and comprising a contact layer (16), a wound filler layer (18), a wound cover membrane (20) and a negative pressure source (21), in which said contact layer (16) is adapted to be located between a wound floor (12) and the abdominal wall (14) and is configured as a contact layer (16) to be in contact with the intestines and other organs in the abdomen, said contact layer (16) comprising at least one inflatable channel (102, 102a, 102b, 102c, 102d, 102e) which is provided with an opening (104) for inflation or deflation of fluid, gel or gas so as to provide a possibility to adjust the stability and rigidity of the contact layer (16), **characterized in that** said contact layer (16) is liquid permeable and associated with the wound filler layer (18) positioned between the contact layer (16) and the negative pressure source (21), said wound filler layer (18) being adapted to provide transport of fluid from the abdominal cavity via said contact layer (16) and via the wound filler layer (18) towards the negative pressure source (21); and said contact layer (16) furthermore being associated with the wound cover membrane (20) which is positioned to fixate the wound filler (18) and provide a seal around the opening in the abdominal wall; and that the contact layer (16) has a planar extension which exceeds the planar extension of the wound filler (18) so that at least a portion of said contact layer (16) is positioned between the wound floor (12) and the abdominal wall (14) during use.

2. The system (2) according to claim 1 **characterized in that** the contact layer comprises at least four inflatable channels.

3. The system (2) according to claim 2 wherein at least some of said inflatable channels (102, 102a, 102b, 102c, 102d, 102e) are designed as oblong channels stretching from the centre portion (108) of the contact layer (16) towards the edges (40) of the contact layer (16).

4. The system (2) according to any of claims 2 or 3 wherein at least some of said inflatable channels (102, 102a, 102b, 102c, 102d, 102e) are designed as circular, oval or asymmetrical channels being circumferentially located around the centre portion (108) of the contact layer (16).

5. The system (2) according to any of claims 2 to 4, wherein all channels (102, 102a, 102b, 102c, 102d, 102e) are connected by communicating conduits and all channels may be (102, 102a, 102b, 102c, 102d, 102e) inflated by introducing fluid through one single opening (104).

6. The system (2) according to any of claims 2 to 4, wherein said channels (102, 102a, 102b, 102c, 102d, 102e) form groups of channels (102a, 102b, 102c, 102d, 102e) such that the channels in each group are connected by communicating conduits and each group of channels may be separately inflated.

7. The system (2) according to claim 6, wherein a first group of channels (102b) is formed on a first half portion of the contact layer and a second group of channels (102c) is formed on a second half portion, each group of channels on each half portion thus being separately inflated.

8. The system (2) according to claim 1 wherein said contact layer (16) comprising one single inflatable channel (102, 102a, 102b, 102c, 102d, 102e).

9. The system (2) according to any one of the preceding claims, wherein said contact layer (16) comprises a top sheet (30) and a bottom sheet (32) and a plurality of inflatable channels (28) located there between.

10. The system (2) according to any one of the preceding claims **characterized in that** the contact layer comprises sealed chambers (28).

## Patentansprüche

1. Unterdruckwundbehandlungssystem (10) für eine offene Wunde der Bauchhöhle und umfassend eine Kontaktschicht (16), eine Wundfüllerschicht (18), eine Wundabdeckmembran (20) und eine Unterdruckquelle (21), wobei die Kontaktschicht (16) angepasst ist, um zwischen einem Wundgrund (12) und der Bauchwand (14) angeordnet zu sein, und konfiguriert ist, um als Kontaktschicht (16) mit dem Darm und anderen Organen in dem Bauch in Kontakt zu stehen, die Kontaktschicht (16) umfassend mindestens einen aufblasbaren Kanal (102, 102a, 102b, 102c, 102d, 102e), der mit einer Öffnung (104) für ein Aufblasen oder ein Entleeren von Fluid, Gel oder Gas versehen ist, um so eine Möglichkeit bereitzustellen, die Stabilität und Steifigkeit der Kontaktschicht (16) zu justieren, **dadurch gekennzeichnet, dass** die Kontaktschicht (16) flüssigkeitsdurchlässig ist und mit der Wundfüllerschicht (18) verknüpft ist, die zwischen der Kontaktschicht (16) und der Unterdruckquelle (21) positioniert ist, wobei die Wundfüllerschicht (18) angepasst ist, um einen Transport von Fluid aus der Bauchhöhle über die Kontaktschicht (16) und über die Wundfüllerschicht (18) zu der Unterdruckquelle (21) bereitzustellen; und wobei die Kontaktschicht (16) ferner mit der Wundabdeckmembran (20) verknüpft ist, die positioniert ist, um den Wundfüller (18) zu fixieren und eine Abdichtung um die Öffnung in der Bauchwand bereitzustellen; und dass die Kontaktschicht (16) eine planare Erstreckung aufweist, die die planare Erstreckung des Wundfüllers (18) übersteigt, so dass mindestens ein Abschnitt der Kontaktschicht (16) während einer Verwendung zwischen dem Wundgrund (12) und der Bauchwand (14) positioniert ist.

2. System (2) nach Anspruch 1, **dadurch gekennzeichnet, dass** die Kontaktschicht mindestens vier aufblasbare Kanäle umfasst.

3. System (2) nach Anspruch 2, wobei mindestens einige der aufblasbaren Kanäle (102, 102a, 102b, 102c, 102d, 102e) als längliche Kanäle gestaltet sind, die sich von dem Mittelabschnitt (108) der Kontaktschicht (16) hin zu den Rändern (40) der Kontaktschicht (16) strecken.

4. System (2) nach einem der Ansprüche 2 oder 3, wobei mindestens einige der aufblasbaren Kanäle (102, 102a, 102b, 102c, 102d, 102e) als kreisförmige, ovale oder asymmetrische Kanäle gestaltet sind, die umlaufend um den Mittelabschnitt (108) der Kontaktschicht (16) angeordnet sind.

5. System (2) nach einem der Ansprüche 2 bis 4, wobei alle Kanäle (102, 102a, 102b, 102c, 102d, 102e) durch kommunizierende Leitungen verbunden sind und alle Kanäle (102, 102a, 102b, 102c, 102d, 102e) durch Einführen von Fluid durch eine einzige Öffnung (104) aufgeblasen werden können.

6. System (2) nach einem der Ansprüche 2 bis 4, wobei die Kanäle (102, 102a, 102b, 102c, 102d, 102e) Gruppen von Kanälen (102a, 102b, 102c, 102d, 102e) ausbilden, derart, dass die Kanäle in jeder Gruppe durch kommunizierende Leitungen verbunden sind und jede Gruppe von Kanälen separat aufgeblasen werden kann.

7. System (2) nach Anspruch 6, wobei eine erste Gruppe von Kanälen (102b) auf einem ersten Halbabschnitt der Kontaktschicht ausgebildet ist und eine zweite Gruppe von Kanälen (102c) auf einem zweiten Halbabschnitt ausgebildet ist, wobei jede Gruppe von Kanälen auf jedem Halbabschnitt somit separat aufgeblasen wird.

8. System (2) nach Anspruch 1, wobei die Kontaktschicht (16) einen einzigen aufblasbaren Kanal (102, 102a, 102b, 102c, 102d, 102e) umfasst.

9. System (2) nach einem der vorstehenden Ansprüche, wobei die Kontaktschicht (16) eine obere Bahn (30) und eine untere Bahn (32) und eine Vielzahl von dazwischen angeordneten aufblasbaren Kanälen (28) umfasst.

10. System (2) nach einem der vorstehenden Ansprüche,
**dadurch gekennzeichnet, dass** die Kontaktschicht abgedichtete Kammern (28) umfasst.

## Revendications

1. Système de traitement de plaies par pression négative (10) pour une plaie ouverte de la cavité abdominale et comprenant une couche de contact (16), une couche de pansement pour cavité de plaie (18), une membrane de couverture de plaie (20) et une source de pression négative (21), dans lequel ladite couche de contact (16) est adaptée pour être située entre un fond de plaie (12) et la paroi abdominale (14) et est conçue comme une couche de contact (16) à entrer en contact avec les intestins et d'autres organes dans l'abdomen, ladite couche de contact (16) comprenant au moins un canal (102, 102a, 102b, 102c, 102d, 102e) gonflable qui est pourvu d'une ouverture (104) pour le gonflage ou le dégonflage de fluide, de gel ou de gaz afin de fournir une possibilité de régler la stabilité et la rigidité de la couche de contact (16), **caractérisé en ce que** ladite couche de contact (16) est perméable aux liquides et associée à la couche de pansement pour cavité de plaie (18) positionnée entre la couche de contact (16) et la source de pression négative (21), ladite couche de pansement pour cavité de plaie (18) étant adaptée pour assurer le transport de fluide à partir de la cavité abdominale par l'intermédiaire de ladite couche de contact (16) et par l'intermédiaire de la couche de pansement pour cavité de plaie (18) vers la source de pression négative (21) ; et ladite couche de contact (16) étant en outre associée à la membrane de couverture de plaie (20) qui est positionnée pour fixer le pansement pour cavité de plaie (18) et assurer une étanchéité autour de l'ouverture dans la paroi abdominale ; et **en ce que** la couche de contact (16) a une extension plane qui dépasse l'extension plane du pansement pour cavité de plaie (18) de sorte qu'au moins une partie de ladite couche de contact (16) soit positionnée entre le fond de plaie (12) et la paroi abdominale (14) pendant l'utilisation.

2. Système (2) selon la revendication 1, **caractérisé en ce que** la couche de contact comprend au moins quatre canaux gonflables.

3. Système (2) selon la revendication 2, dans lequel au moins certains desdits canaux (102, 102a, 102b, 102c, 102d, 102e) gonflables sont conçus comme des canaux oblongs s'étendant à partir de la partie centrale (108) de la couche de contact (16) vers les bords (40) de la couche de contact (16).

4. Système (2) selon l'une quelconque des revendications 2 ou 3, dans lequel au moins certains desdits canaux (102, 102a, 102b, 102c, 102d, 102e) gonflables sont des canaux circulaires, ovales ou asymétriques qui sont situés circonférentiellement autour de la partie centrale (108) de la couche de contact (16).

5. Système (2) selon l'une quelconque des revendications 2 à 4, dans lequel tous les canaux (102, 102a, 102b, 102c, 102d, 102e) sont reliés par des conduits de communication et tous les canaux (102, 102a, 102b, 102c, 102d, 102e) peuvent être gonflés en introduisant un fluide à travers une ouverture (104) unique.

6. Système (2) selon l'une quelconque des revendications 2 à 4, dans lequel lesdits canaux (102, 102a, 102b, 102c, 102d, 102e) forment des groupes de canaux (102a, 102b, 102c, 102d, 102e) de telle sorte que les canaux de chaque groupe soient reliés par des conduits de communication et que chaque groupe de canaux puisse être gonflé séparément.

7. Système (2) selon la revendication 6, dans lequel un premier groupe de canaux (102b) est formé sur une première moitié de la couche de contact et un second groupe de canaux (102c) est formé sur une seconde moitié, chaque groupe de canaux sur chaque moitié étant ainsi gonflé séparément.

8. Système (2) selon la revendication 1, dans lequel ladite couche de contact (16) comprenant un seul canal (102, 102a, 102b, 102c, 102d, 102e) gonflable.

9. Système (2) selon l'une quelconque des revendications précédentes, dans lequel ladite couche de contact (16) comprend une feuille supérieure (30) et une feuille inférieure (32) et une pluralité de canaux (28) gonflables situés entre celles-ci.

10. Système (2) selon l'une quelconque des revendications précédentes, **caractérisé en ce que** la couche de contact comprend des chambres (28) scellées.
